# EUROPEAN PATENT APPLICATION

(11) **EP 4 807 367 A2**
(43) Date of publication of application: **16.09.2026**
(21) Application number: 26183061.6
(22) Date of filing: 25.02.2021
(51) Int. Cl.: G01N 33/566

(54) **ENZYMES FOR SIALYLATION OF GLYCANS**

(30) Priority: 25.02.2020 US 202062981293 P; 19.05.2020 US 202063026927 P
(62) Document number(s) of the earlier application(s) in accordance with Art. 76 EPC: 21760787.8 / 4 110 391
(71) Applicant: Momenta Pharmaceuticals, Inc., Titusville, NJ 08560 (US)
(72) Inventor: MEADOR III, James, Framingham, MA 01702 (US); SIPSEY, Sandra Freitas Pavao, Horsham, PA 19044 (US); MEDEIROS, Amy, Titusville, NJ 08560 (US); GURNANI, Srishti, Titusville, NJ 08560 (US)
(74) Representative: Mathys & Squire

(57) **Abstract**

Described herein are fusion proteins, e.g., fusion proteins comprising enzymatically active portion(s) of ST6Gall or B4GalT1 as well as methods for producing them, nucleic acid molecule(s) encoding the fusion protein(s), vectors comprising the nucleic acid molecule(s), and host cell(s) comprising the vector(s). Also described herein are methods of sialyating immunoglobulin G (IgG) antibodies.

## Description

### CLAIM OF PRIORITY

This application claims the benefit of U.S. Provisional Application Serial No. 62/981,293, filed on February 25, 2020, and U.S. Provisional Application Serial No. 63/026,927, filed on May 19, 2020. The entire contents of the foregoing are incorporated herein by reference.

### TECHNICAL FIELD

The present disclosure relates to fusion proteins, e.g., fusion proteins comprising enzymatically active portion(s) of ST6Gal1 or B4GalT1 as well as methods for producing them, nucleic acid molecule(s) encoding the fusion protein(s), vectors comprising the nucleic acid molecule(s), and host cell(s) comprising the vector(s). Also described herein are methods of sialyating immunoglobulin G (IgG) antibodies.

### BACKGROUND

Intravenous immunoglobulin (IVIg), which is prepared from the pooled plasma of human donors (e.g., pooled plasma from at least 1,000 donors), is used to treat a variety of inflammatory disorders. However, IVIg preparations have distinct limitations, such as variable efficacy, clinical risks, high costs, and finite supply. Different IVIg preparations are frequently treated as interchangeable products clinically, but it is well-known that significant differences in product preparations exist that may impact tolerability and activity in selected clinical applications. At the current maximal dosing regimens, only partial and unsustained responses are obtained in many instances. In addition, the long infusion times (4-6 h) associated with the high volume of IVIg treatment consume significant resources at infusion centers and negatively affect patient-reported outcomes, such as convenience and quality of life.

The identification of the important anti-inflammatory role of Fc domain sialylation has presented an opportunity to develop more potent immunoglobulin therapies. Commercially available IVIg preparations generally exhibit low levels of sialylation on the Fc domain of the antibodies present. Specifically, they exhibit low levels of di-sialylation of the branched glycans on the Fc region.

Washburn et al. (Proceedings of the National Academy of Sciences, USA 112:E1297-E1306 (2015)) describes a controlled sialylation process to generate highly tetra-Fc-sialylated IVIg and showed that the process yields a product with consistent enhanced anti-inflammatory activity.

### SUMMARY

Described herein are methods for preparing immunoglobulin G (IgG) having a very high level of Fc sialylation. The methods described herein can provide hypersialylated IgG (hsIgG) in which greater than 70% of the branched glycans on the Fc domain are sialylated on both branches (i.e., on the alpha 1,3 branch and on the alpha 1,6 branch). HsIgG contains a diverse mixture of IgG antibody subtypes with IgG1 antibodies being most prevalent followed by IgG2. The diversity of the antibodies is very high because the starting material is IgG antibodies pooled from many hundreds or several thousand donors. The IgG antibodies used to prepare hsIgG can be obtained, for example from pooled human plasma (e.g., pooled plasma from at least 1,000-30,000 donors). Alternatively, IVIg, including commercially available IVIg, can be used to prepared hsIgG. HsIgG has far higher level of sialic acid on the branched glycans on the Fc region than does IVIg. This results in a composition that differs from IVIg in both structure and activity. HsIgG can be prepared as described in WO2014/179601 or Washburn et al. (Proceedings of the National Academy of Sciences, USA 112: E1297-E1306 (2015)), both of which are hereby incorporated by reference.

Described herein are improved methods for preparing hsIgG.

In hypersialylated IgG at least 60% (e.g., 65%, 70%, 75%, 80%, 82%, 85%, 87%, 90%, 92%, 94%, 95%, 97%, 98% up to and including 100%) of branched glycans on the Fc region are di-sialylated (i.e., on both the α 1,3 branch and the α 1,6 arm) by way of NeuAc-α 2,6-Gal terminal linkages. In some embodiments, less than 50% (e.g., less than 40%, 30%, 20%, 15%, 10%, 5%, 4%, 3%, 2%, 1%) of branched glycans on the Fc region are mono-sialylated (i.e., sialylated only on the α 1,3 branch or only on the α 1,6 branch) by way of a NeuAc-α 2,6-Gal terminal linkage.

In some embodiments, the polypeptides are derived from plasma, e.g., human plasma. In certain embodiments, the polypeptides are overwhelmingly IgG polypeptides (e.g., IgG1, IgG2, IgG3 or IgG4 or mixtures thereof), although trace amounts of other contain trace amount of other immunoglobulin subclasses can be present.

As used herein, the term "antibody" refers to a polypeptide that includes at least one immunoglobulin variable region, e.g., an amino acid sequence that provides an immunoglobulin variable domain or immunoglobulin variable domain sequence. For example, an antibody can include a heavy (H) chain variable region (abbreviated herein as V_{H}), and a light (L) chain variable region (abbreviated herein as V_{L}). In another example, an antibody includes two heavy (H) chain variable regions and two light (L) chain variable regions. The term "antibody" encompasses antigen-binding fragments of antibodies (e.g., single chain antibodies, Fab, F(ab')₂, Fd, Fv, and dAb fragments) as well as complete antibodies, e.g., intact immunoglobulins of types IgA, IgG, IgE, IgD, IgM (as well as subtypes thereof). The light chains of the immunoglobulin can be of types kappa or lambda.

As used herein, the term "constant region" refers to a polypeptide that corresponds to, or is derived from, one or more constant region immunoglobulin domains of an antibody. A constant region can include any or all of the following immunoglobulin domains: a C_{H}1 domain, a hinge region, a C_{H}2 domain, a C_{H}3 domain (derived from an IgA, IgD, IgG, IgE, or IgM), and a C_{H}4 domain (derived from an IgE or IgM).

As used herein, the term "Fc region" refers to a dimer of two "Fc polypeptides," each "Fc polypeptide" including the constant region of an antibody excluding the first constant region immunoglobulin domain. In some embodiments, an "Fc region" includes two Fc polypeptides linked by one or more disulfide bonds, chemical linkers, or peptide linkers. "Fc polypeptide" refers to the last two constant region immunoglobulin domains of IgA, IgD, and IgG, and the last three constant region immunoglobulin domains of IgE and IgM, and may also include part or the entire flexible hinge N-terminal to these domains. For IgG, "Fc polypeptide" comprises immunoglobulin domains Cgamma2 (Cy2) and Cgamma3 (Cy3) and the lower part of the hinge between Cgamma1 (Cγ1) and Cy2. Although the boundaries of the Fc polypeptide may vary, the human IgG heavy chain Fc polypeptide is usually defined to comprise residues starting at T223 or C226 or P230, to its carboxyl-terminus, wherein the numbering is according to the EU index as in Kabat et al. (1991, NIH Publication 91-3242, National Technical Information Services, Springfield, VA). For IgA, Fc polypeptide comprises immunoglobulin domains Calpha2 (Cα2) and Calpha3 (Cα3) and the lower part of the hinge between Calpha1 (Cα1) and Cα2. An Fc region can be synthetic, recombinant, or generated from natural sources such as IVIg.

As used herein, "glycan" is a sugar, which can be monomers or polymers of sugar residues, such as at least three sugars, and can be linear or branched. A "glycan" can include natural sugar residues (e.g., glucose, N-acetylglucosamine, N-acetyl neuraminic acid, galactose, mannose, fucose, hexose, arabinose, ribose, xylose, etc.) and/or modified sugars (e.g., 2'-fluororibose, 2'-deoxyribose, phosphomannose, 6'sulfo N-acetylglucosamine, etc.). The term "glycan" includes homo and heteropolymers of sugar residues. The term "glycan" also encompasses a glycan component of a glycoconjugate (e.g., of a polypeptide, glycolipid, proteoglycan, etc.). The term also encompasses free glycans, including glycans that have been cleaved or otherwise released from a glycoconjugate.

As used herein, the term "glycoprotein" refers to a protein that contains a peptide backbone covalently linked to one or more sugar moieties (i.e., glycans). The sugar moiety(ies) may be in the form of monosaccharides, disaccharides, oligosaccharides, and/or polysaccharides. The sugar moiety(ies) may comprise a single unbranched chain of sugar residues or may comprise one or more branched chains. Glycoproteins can contain O-linked sugar moieties and/or N-linked sugar moieties.

As used herein, "IVIg" is a preparation of pooled, polyvalent IgG, including all four IgG subgroups, extracted from plasma of at least 1,000 human donors. IVIg is approved as a plasma protein replacement therapy for immune deficient patients. The level of IVIg Fc glycan sialylation varies among IVIg preparations, but is generally less than 20%. The level of disialylation is generally far lower. As used herein, the term "derived from IVIg" refers to polypeptides which result from manipulation of IVIg. For example, polypeptides purified from IVIg (e.g., enriched for sialylated IgGs or modified IVIg (e.g., IVIg IgGs enzymatically sialylated).

As used herein, an "N-glycosylation site of an Fc polypeptide" refers to an amino acid residue within an Fc polypeptide to which a glycan is N-linked. In some embodiments, an Fc region contains a dimer of Fc polypeptides, and the Fc region comprises two N-glycosylation sites, one on each Fc polypeptide.

As used herein "percent (%) of branched glycans" refers to the number of moles of glycan X relative to total moles of glycans present, wherein X represents the glycan of interest.

The term "pharmaceutically effective amount" or "therapeutically effective amount" refers to an amount (e.g., dose) effective in treating a patient, having a disorder or condition described herein. It is also to be understood herein that a "pharmaceutically effective amount" may be interpreted as an amount giving a desired therapeutic effect, either taken in one dose or in any dosage or route, taken alone or in combination with other therapeutic agents.

"Pharmaceutical preparations" and "pharmaceutical products" can be included in kits containing the preparation or product and instructions for use.

"Pharmaceutical preparations" and "pharmaceutical products" generally refer to compositions in which the final predetermined level of sialylation has been achieved, and which are free of process impurities. To that end, "pharmaceutical preparations" and "pharmaceutical products" are substantially free of ST6Gal sialyltransferase and/or sialic acid donor (e.g., cytidine 5'-monophospho-N-acetyl neuraminic acid) or the byproducts thereof (e.g., cytidine 5'-monophosphate).

"Pharmaceutical preparations" and "pharmaceutical products" are generally substantially free of other components of a cell in which the glycoproteins were produced (e.g., the endoplasmic reticulum or cytoplasmic proteins and RNA), if recombinant.

By "purified" (or "isolated") refers to a polynucleotide or a polypeptide that is removed or separated from other components present in its natural environment. For example, an isolated polypeptide is one that is separated from other components of a cell in which it was produced (e.g., the endoplasmic reticulum or cytoplasmic proteins and RNA). An isolated polynucleotide is one that is separated from other nuclear components (e.g., histones) and/or from upstream or downstream nucleic acids. An isolated polynucleotide or polypeptide can be at least 60% free, or at least 75% free, or at least 90% free, or at least 95% free from other components present in natural environment of the indicated polynucleotide or polypeptide.

As used herein, the term "sialylated" refers to a glycan having a terminal sialic acid. The term "mono-sialylated" refers to branched glycans having one terminal sialic acid, e.g., on an α1,3 branch or an α1,6 branch. The term "di-sialylated" refers to a branched glycan having a terminal sialic acid on two arms, e.g., both an α1,3 arm and an α1,6 arm.

Provided here is a fusion protein comprising: an N-terminal signal sequence; and an enzymatically active portion of human Alpha-2,6-sialyltransferase 1 (ST6Gal1).

In some embodiments, the enzymatically active portion of ST6Gal1 comprises **SEQ ID NO:4.** In some embodiments, the enzymatically active portion of ST6Gal1 consists of **SEQ ID NO:4.**

In some embodiments, the signal sequence is an N-terminal azurocidin signal sequence. In some embodiments, the azurocidin signal sequence comprises MTRLTVLALLAGLLASSRA (**SEQ ID NO:30**). In some embodiments, the azurocidin signal sequence consists of MTRLTVLALLAGLLASSRA (**SEQ ID NO:30**).

In some embodiments, the fusion protein further comprises an affinity tag.

In some embodiments, the affinity tag is selected from the group consisting of polyhistidine, glutathione S-transferase (GST), maltose-binding protein (MBP), chitin binding protein, a streptavidin tag (e.g., Trp-Ser-His-Pro-Gln-Phe-Glu-Lys (**SEQ ID NO:31**)), FLAG-tag (e.g., DYKDDDDK (**SEQ ID NO:32**)), a biotin tag, and combinations thereof.

In some embodiments, the polyhistidine tag is selected from the group consisting of HHHH (**SEQ ID NO:11**), HHHHH (**SEQ ID NO:12**), HHHHHH, (**SEQ ID NO:13**), HHHHHHH (**SEQ ID NO:14**), HHHHHHHH (**SEQ ID NO:15**), HHHHHHHHH (**SEQ ID NO:16**), and HHHHHHHHHH (**SEQ ID NO:17**).

In some embodiments, the affinity tag is situated towards the N-terminal side of the enzymatically active portion of ST6Gal1.

In some embodiments, the N-terminal signal sequence comprises MTRLTVLALLAGLLASSRA (**SEQ ID NO:30**), and the enzymatically active portion of ST6Gal1 comprises **SEQ ID NO:4.**

In some embodiments, the fusion protein further comprises a hexahistidine tag. In some embodiments, the hexahistidine tag is between the N-terminal signal sequence and the enzymatically active portion of ST6Gal1. In some embodiments, the fusion protein consists of

### SEQ ID NO:6.

Also provided herein are nucleic acid molecule(s) encoding the fusion protein(s), vector(s) comprising the nucleic acid molecule(s), and host cell(s) transformed, preferably stably, with the vector(s).

In some embodiments, the vector further comprises a promoter operably linked to the nucleic acid encoding the fusion protein. In some embodiments, the promoter is a cytomegalovirus (CMV) promoter.

In some embodiments, the host cell is a human embryonic kidney (HEK) cell, or derivative thereof. In some embodiments, the host cell is the HEK derivative HEK293.

Also provided herein is a method for producing a polypeptide comprising: culturing the host cell as described herein in a culture medium under conditions permissive for expression of the fusion protein; and isolating the fusion protein from the culture medium.

Also provided herein is a fusion protein comprising: an N-terminal signal sequence; and an enzymatically active portion of human beta-1,4-galactosyltransferase (B4GalT1).

In some embodiments, the enzymatically active portion of B4GalT1 comprises **SEQ ID NO:43.** In some embodiments, the enzymatically active portion of B4GalT1 consists of **SEQ ID NO:43.**

In some embodiments, the signal sequence is an N-terminal azurocidin signal sequence. In some embodiments, the azurocidin signal sequence comprises MTRLTVLALLAGLLASSRA (**SEQ ID NO:30**). In some embodiments, the azurocidin signal sequence consists of MTRLTVLALLAGLLASSRA (**SEQ ID NO:30**).

In some embodiments, the fusion protein further comprises an affinity tag.

In some embodiments, the affinity tag is selected from the group consisting of polyhistidine, glutathione S-transferase (GST), maltose-binding protein (MBP), chitin binding protein, a streptavidin tag (e.g., Trp-Ser-His-Pro-Gln-Phe-Glu-Lys (**SEQ ID NO:31**)), FLAG-tag (e.g., DYKDDDDK (**SEQ ID NO:32**)), a biotin tag, and combinations thereof.

In some embodiments, the polyhistidine tag is selected from the group consisting of HHHH (**SEQ ID NO:11**), HHHHH (**SEQ ID NO:12**), HHHHHH, (**SEQ ID NO:13**), HHHHHHH (**SEQ ID NO:14**), HHHHHHHH (**SEQ ID NO:15**), HHHHHHHHH (**SEQ ID NO:16**), and HHHHHHHHHH (**SEQ ID NO:17**).

In some embodiments, the affinity tag is situated towards the C-terminal side of the enzymatically active portion of B4GalT1.

In some embodiments, the N-terminal signal sequence comprises MTRLTVLALLAGLLASSRA (**SEQ ID NO:30**), and the enzymatically active portion of B4GalT1comprises **SEQ ID NO:43.**

In some embodiments, the fusion protein further comprises a septahistidine tag. In some embodiments, the septahistidine tag is C-terminal.

In some embodiments, the fusion protein consists of **SEQ ID NO:45.**

Also provided herein are nucleic acid molecule(s) encoding the fusion protein(s), vector(s) comprising the nucleic acid molecule(s), and host cell(s) transformed, preferably stably, with the vector(s).

In some embodiments, the vector further comprises a promoter operably linked to the nucleic acid encoding the fusion protein. In some embodiments, the promoter is a cytomegalovirus (CMV) promoter.

In some embodiments, the host cell is a human embryonic kidney (HEK) cell, or derivative thereof. In some embodiments, the host cell is the HEK derivative HEK293.

Also provided herein is a method for producing a polypeptide comprising: culturing the host cell as described herein in a culture medium under conditions permissive for expression of the fusion protein; and isolating the fusion protein from the culture medium.

Also provided herein is a method for sialyating immunoglobulin G (IgG) antibodies comprising:
a) providing a composition comprising IgG antibodies;
b) exposing the composition to a β1,4-galactosyltransferase 1 and an enzymatically active portion of ST6Gal1 comprising **SEQ ID NO:4** in the presence of UDP-Gal and CMP-NANA, thereby producing a composition comprising sialyated IgG (sIgG).

Also provided herein is a method for sialyating immunoglobulin G (IgG) antibodies comprising: a) providing a composition comprising IgG antibodies; b) exposing the IgG antibodies to a β1,4-galactosyltransferase 1 in the presence of UDP-Gal, thereby producing a composition comprising galactosylated IgG antibodies; and c) exposing the composition comprising galactosylated IgG antibodies to an enzymatically active portion of ST6Gal1 comprising **SEQ ID NO:4** in the presence of CMP-NANA, thereby producing a composition comprising sialyated IgG (sIgG).

In some embodiments, the composition comprising galactosylated IgG antibodies is not purified prior to step (c).

In some embodiments, the method further comprises supplementing one or more of the compositions with CMP-NANA.

In some embodiments, the mixture of IgG antibodies are selected from the group consisting of IgG1, IgG2, IgG3, IgG4, and combinations thereof.

In some embodiments, at least 60% of branched glycans on the Fc region of the antibodies in the composition comprising sIgG are di-sialylated.

In some embodiments, less than 50% of branched glycans on the Fc regions of the antibodies in the composition comprising sIgG are mono-sialylated.

Provided herein is a human embryonic kidney (HEK) cell stably transformed with a nucleic acid molecule comprising a nucleic acid sequence encoding fusion protein comprising an azurocidin signal sequence and a portion of human ST6 sialyltransferase consisting of **SEQ ID NO:4.**

In some embodiments, the fusion protein comprises a sequence selected from HHHHH (**SEQ ID NO:12**), HHHHHH (**SEQ ID NO:13**), HHHHHHH (**SEQ ID NO:14**), HHHHHHHH (**SEQ ID NO:15**), HHHHHHHHH (**SEQ ID NO:16**), HHHHHHHHHH (**SEQ ID NO:17**), HHHHHM (**SEQ ID NO:18**), HHHHHHM (**SEQ ID NO:19**), HHHHHHM (**SEQ ID NO:20**), HHHHHHHHM (**SEQ ID NO:21**), HHHHHHHHHM (**SEQ ID NO:22**), and HHHHHHHHHHM (**SEQ ID NO:23**) located between the azuricidin signal sequence the portion of human ST6 sialyltransferase consisting of **SEQ ID NO:4.**

In some embodiments, the fusion protein lacks the portion of human ST6 sialyltransferase amino terminal to **SEQ ID NO:4.**

In some embodiments, the fusion protein comprises SEQ ID NO:4, but lacks the portion of human ST6 sialyltransferase amino terminal to **SEQ ID NO:4.**

In some embodiments, the fusion protein comprises the amino acid sequence of **SEQ ID NO:6.** In some embodiments, the fusion protein comprises the amino acid sequence of **SEQ ID NO:3.**

In some embodiments, the nucleic acid molecule comprises a promoter operably linked to the nucleic acid sequence encoding the fusion protein. In some embodiments, the promoter is a cytomegalovirus promoter.

Also provided herein is a method for preparing a polypeptide comprising **SEQ ID NO:3** comprising culturing the HEK cells in culture medium under conditions permissive for expression of the fusion protein and isolating a polypeptide comprising **SEQ ID NO:3** from the culture medium.

In some embodiments, the method further comprises purifying to at least 95% w/w the isolated polypeptide.

Also provided herein is a polypeptide comprising **SEQ ID NO:3** or **SEQ ID NO:6.**

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention will be apparent from the following detailed description and figures, and from the claims.

### DESCRIPTION OF DRAWINGS

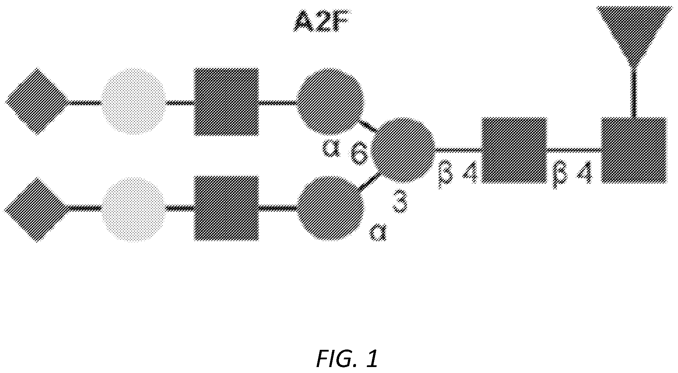

**FIG. 1** shows a short, branched core oligosaccharide comprising two N-acetylglucosamine and three mannose residues. One of the branches is referred to in the art as the "α 1,3 arm," and the second branch is referred to as the "α 1,6 arm,". Squares: N-acetylglucosamine; dark gray circles: mannose; light gray circles: galactose; diamonds: N-acetylneuraminic acid; triangles: fucose.

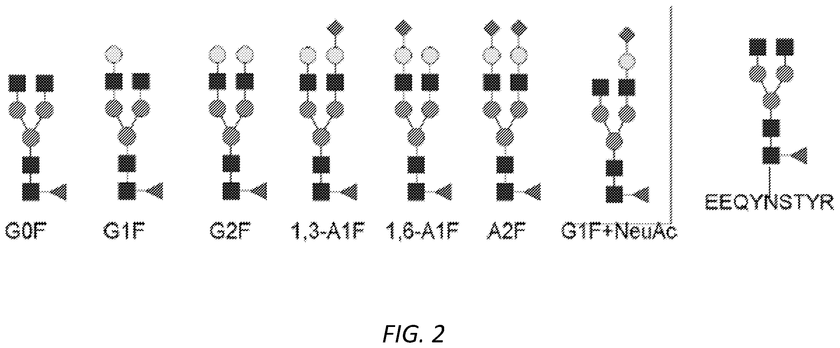

**FIG. 2** shows common Fc glycans present in IVIg. Squares: N-acetylglucosamine; dark gray circles: mannose; light gray circles: galactose; diamonds: N-acetylneuraminic acid; triangles: fucose.

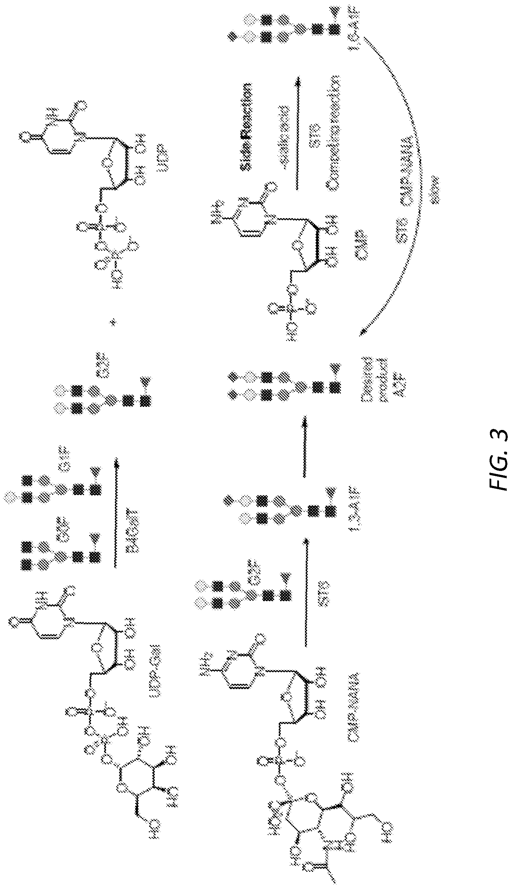

**FIG. 3** shows how immunoglobulins, e.g., IgG antibodies, can be sialylated by carrying out a galactosylation step followed by a sialylation step. Squares: N-acetylglucosamine; dark gray circles: mannose; light gray circles: galactose; diamonds: N-acetylneuraminic acid; triangles: fucose.

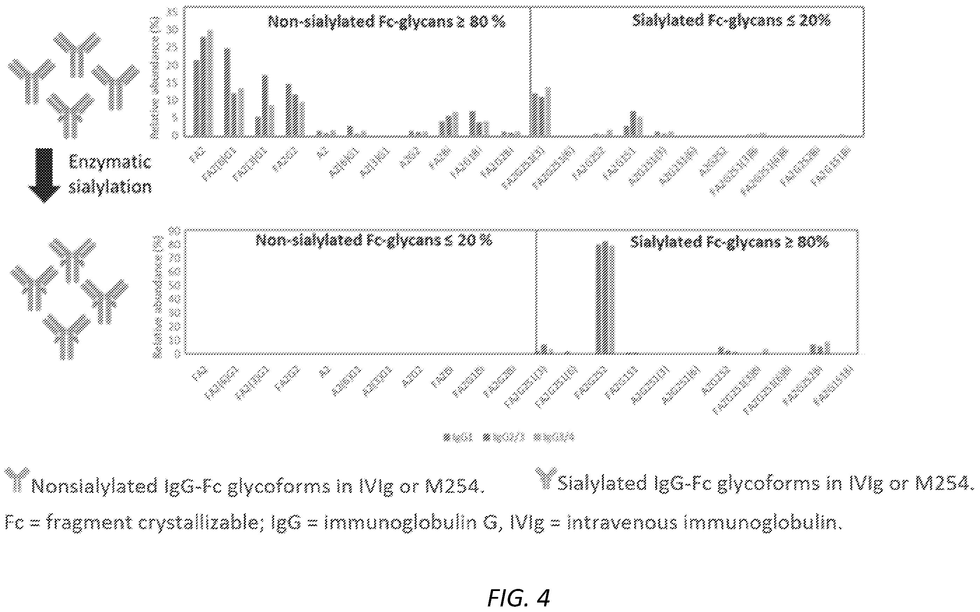

**FIG. 4** shows the reaction product of a representative example of the IgG-Fc glycan profile for a reaction starting with IVIg. The the left panel is a schematic representation of enzymatic sialylation reaction to transform IgG to hsIgG; the right panel is the IgG Fc glycan profile for the starting IVIg and hsIgG. Bars, from left to right, correspond to IgG1, IgG2/3, and IgG3/4, respectively.

### DETAILED DESCRIPTION

Antibodies are glycosylated at conserved positions in the constant regions of their heavy chain and within the Fab. For example, within the Fc domain, human IgG antibodies have a single N-linked glycosylation site at Asn297 of the CH2 domain. Each antibody isotype has a distinct variety of N-linked carbohydrate structures in the constant regions. For human IgG, the core oligosaccharide normally consists of GlcNAc₂Man₃GlcNAc, with differing numbers of outer residues. Variation among individual IgG's can occur via attachment of galactose and/or galactose-sialic acid at one or both terminal GlcNAc or via attachment of a third GlcNAc arm (bisecting GlcNAc).

The present disclosure encompasses, in part, methods for preparing immunoglobulins (e.g., human IgG) having an Fc region having particular levels of branched glycans that are sialylated on both of the arms of the branched glycan (e.g., with a NeuAc-α 2,6-Gal terminal linkage). The levels can be measured on an individual Fc region (e.g., the number of branched glycans that are sialylated on an α1,3 arm, an α1,6 arm, or both, of the branched glycans in the Fc region), or on the overall composition of a preparation of polypeptides (e.g., the number or percentage of branched glycans that are sialylated on an α1,3 arm, an α1,6 arm, or both, of the branched glycans in the Fc region in a preparation of polypeptides).

Naturally derived polypeptides that can be used to prepare hypersialylated IgG include, for example, IgG in human serum (particular human serum pooled from more than 1,000 donors), intravenous immunoglobulin (IVIg) and polypeptides derived from IVIg (e.g., polypeptides purified from IVIg (e.g., enriched for sialylated IgGs) or modified IVIg (e.g., IVIg IgGs enzymatically sialylated).

N-linked oligosaccharide chains are added to a protein in the lumen of the endoplasmic reticulum. Specifically, an initial oligosaccharide (typically 14-sugar) is added to the amino group on the side chain of an asparagine residue contained within the target consensus sequence of Asn-X-Ser/Thr, where X may be any amino acid except proline. The structure of this initial oligosaccharide is common to most eukaryotes, and contains three glucose, nine mannose, and two N-acetylglucosamine residues. This initial oligosaccharide chain can be trimmed by specific glycosidase enzymes in the endoplasmic reticulum, resulting in a short, branched core oligosaccharide composed of two N-acetylglucosamine and three mannose residues. One of the branches is referred to in the art as the "α 1,3 arm," and the second branch is referred to as the "α 1,6 arm," as shown in **FIG. 1****.**

N-glycans can be subdivided into three distinct groups called "high mannose type," "hybrid type," and "complex type," with a common pentasaccharide core (Man (α 1,6)-(Man(α 1,3))-Man(β 1,4)-GlcpNAc(β 1,4)-GlcpNAc(β 1,N)-Asn) occurring in all three groups.

The more common Fc glycans present in IVIg are shown in **FIG. 2****.**

Additionally or alternatively, one or more monosaccharides units of N-acetylglucosamine may be added to the core mannose subunits to form a "complex glycan." Galactose may be added to the N-acetylglucosamine subunits, and sialic acid subunits may be added to the galactose subunits, resulting in chains that terminate with any of a sialic acid, a galactose or an N-acetylglucosamine residue. Additionally, a fucose residue may be added to an N-acetylglucosamine residue of the core oligosaccharide. Each of these additions is catalyzed by specific glycosyl transferases.

"Hybrid glycans" comprise characteristics of both high-mannose and complex glycans. For example, one branch of a hybrid glycan may comprise primarily or exclusively mannose residues, while another branch may comprise N-acetylglucosamine, sialic acid, galactose, and/or fucose sugars.

Sialic acids are a family of 9-carbon monosaccharides with heterocyclic ring structures. They bear a negative charge via a carboxylic acid group attached to the ring as well as other chemical decorations including N-acetyl and N-glycolyl groups. The two main types of sialyl residues found in polypeptides produced in mammalian expression systems are N-acetylneuraminic acid (NeuAc) and N-glycolylneuraminic acid (NeuGc). These usually occur as terminal structures attached to galactose (Gal) residues at the non-reducing termini of both N- and O-linked glycans. The glycosidic linkage configurations for these sialyl groups can be either α 2,3 or α 2,6.

Fc regions are glycosylated at conserved, N-linked glycosylation sites. For example, each heavy chain of an IgG antibody has a single N-linked glycosylation site at Asn297 of the C_{H}2 domain. IgA antibodies have N-linked glycosylation sites within the C_{H}2 and C_{H}3 domains, IgE antibodies have N-linked glycosylation sites within the C_{H}3 domain, and IgM antibodies have N-linked glycosylation sites within the C_{H}1, C_{H}2, C_{H}3, and C_{H}4 domains.

Each antibody isotype has a distinct variety of N-linked carbohydrate structures in the constant regions. For example, IgG has a single N-linked biantennary carbohydrate at Asn297 of the C_{H}2 domain in each Fc polypeptide of the Fc region, which also contains the binding sites for C1q and FcyR. For human IgG, the core oligosaccharide normally consists of GlcNAc₂Man₃GlcNAc, with differing numbers of outer residues. Variation among individual IgG can occur via attachment of galactose and/or galactose-sialic acid at one or both terminal GlcNAc or via attachment of a third GlcNAc arm (bisecting GlcNAc).

Immunoglobulins, e.g., IgG antibodies, can be sialylated by carrying out a galactosylation step followed by a sialylation step. Beta-1,4-galactosyltransferase 1 (B4GalT) is a Type II Golgi membrane-bound glycoprotein that transfers galactose from uridine 5'-diphosphosegalactose ([[(2*R*,3*S*,4*R*,5*R*)-5-(2,4-dioxopyrimidin-1-yl)-3,4-dihydroxyoxolan-2-yl]methoxy-hydroxyphosphoryl] [(2*R*,3*R*,4*R*,5*R*,6*R*)-3,4,5-trihydroxy-6-(hydroxymethyl)oxan-2-yl] hydrogen phosphate; **UDP-Gal**) to GlcNAc as a β-1,4 linkage. Alpha-2,6-sialyltransferase 1 (ST6) is a Type II Golgi membrane-bound glycoprotein that transfers sialic acid from cytidine 5'-monophospho-Nacetylneuraminicacid ((2*R*,4*S*,5*R*,6*R*)-5-acetamido-2-[[(2*R*,3*S*,4*R*,5*R*)-5-(4-amino-2-oxopyrimidin-1-yl)-3,4-dihydroxyoxolan-2-yl]methoxy-hydroxyphosphoryl]oxy-4-hydroxy-6-(1,2,3-trihydroxypropyl)oxane-2-carboxylic acid; **CMP-NANA** or **CMP-Sialic Acid**) to Gal as an α-2,6 linkage. Schematically, the reactions proceed shown in **FIG. 3****.**

Glycans of polypeptides can be evaluated using any methods known in the art. For example, sialylation of glycan compositions (e.g., level of branched glycans that are sialylated on an α1,3 branch and/or an α1,6 branch) can be characterized using methods described in WO2014/179601.

In some embodiments of the hsIgG compositions prepared by the methods described herein, at least 60%, 65%, 70%, 75%, 80%, 85%, or 90% of the branched glycans on the Fc domain have a sialic acid on both the α 1,3 arm and the α 1,6 arm that is connected through a NeuAc-α 2,6-Gal terminal linkage. In addition, in some embodiments, at least 40%, 50%, 60%, 65%, 70%, 75%, 80%, or 85% of the branched glycans on the Fab domain have a sialic acid on both the α 1,3 arm and the α 1,6 arm that is connected through a NeuAc-α 2,6-Gal terminal linkage. Overall, in some embodiments, at least 60%, 65%, 70%, 75%, 80%, 85%, or 90% of the branched glycans have a sialic acid on both the α 1,3 arm and the α 1,6 arm that is connected through a NeuAc-α 2,6-Gal terminal linkage.

### Enzymes

Beta-1,4-galactosyltransferase (B4GalT), e.g., human B4GalT, e.g., human B4Galt1, as well as orthologs, mutants, and variants thereof, including enzymatically active portions of beta-1,4-galactosyltransferase (B4GalT), e.g., human B4GalT, e.g., human B4Galt1, as well as orthologs, mutants, and variants thereof, and fusion proteins comprising the same are suitable for use in the methods described herein. B4Galt1 is one of seven beta-1,4-galactosyltransferase

(beta4GalT) genes that each encode type II membrane-bound glycoproteins that appear to have exclusive specificity for the donor substrate UDP-galactose; all transfer galactose in a beta1,4 linkage to similar acceptor sugars: GlcNAc, Glc, and Xyl. B4Galt1 adds galactose to N-acetylglucosamine residues that are either monosaccharides or the nonreducing ends of glycoprotein carbohydrate chains. B4GalT1 is also called GGTB2. Four alternative transcripts encoding four isoforms of B4GALT1 (NCBI Gene ID 2683) are described in **Table 1.**

**Table 1. Human B4GALT1 isoforms**

| Transcript | Length (nt) | Protein | **SEQ ID NO:** | Length (aa) | Isoform |
|---|---|---|---|---|---|
| NM_001497.4 | 4176 | NP_001488.2 | 37 | 398 | 1 |
| NM_001378495.1 | 3999 | NP_001365424.1 | 38 | 385 | 2 |
| NM_001378496.1 | 4053 | NP_001365425.1 | 39 | 357 | 3 |
| NM_001378497.1 | 1520 | NP_001365426.1 | 40 | 225 | 4 |

>NP_001488.2 B4GALT1 [organism=Homo sapiens] [GeneID=2683] [isoform=1] (**SEQ ID NO:37**) 
MRLREPLLSGSAAMPGASLQRACRLLVAVCALHLGVTLVYYLAGRDLSRLPQLVGVSTPLQGGS
NSAAAIGQSSGELRTGGARPPPPLGASSQPRPGGDSSPVVDSGPGPASNLTSVPVPHTTALSLP
ACPEESPLLVGPMLIEFNMPVDLELVAKQNPNVKMGGRYAPRDCVSPHKVAIIIPFRNRQEHLK
YWLYYLHPVLQRQQLDYGIYVINQAGDTIFNRAKLLNVGFQEALKDYDYTCFVFSDVDLIPMND
HNAYRCFSQPRHISVAMDKFGFSLPYVQYFGGVSALSKQQFLTINGFPNNYWGWGGEDDDIFNR
LVFRGMSISRPNAVVGRCRMIRHSRDKKNEPNPQRFDRIAHTKETMLSDGLNSLTYQVLDVQRY
PLYTQITVDIGTPS
>NP_001365424.1 B4GALT1 [organism=Homo sapiens] [GeneID=2683] [isoform=2] (**SEQ ID NO:38**) 
MPGASLQRACRLLVAVCALHLGVTLVYYLAGRDLSRLPQLVGVSTPLQGGSNSAAAIGQSSGEL
RTGGARPPPPLGASSQPRPGGDSSPVVDSGPGPASNLTSVPVPHTTALSLPACPEESPLLVGPM
LIEFNMPVDLELVAKQNPNVKMGGRYAPRDCVSPHKVAIIIPFRNRQEHLKYWLYYLHPVLQRQ
QLDYGIYVINQAGDTIFNRAKLLNVGFQEALKDYDYTCFVFSDVDLIPMNDHNAYRCFSQPRHI
SVAMDKFGFSLPYVQYFGGVSALSKQQFLTINGFPNNYWGWGGEDDDIFNRLVFRGMSISRPNA
VVGRCRMIRHSRDKKNEPNPQRFDRIAHTKETMLSDGLNSLTYQVLDVQRYPLYTQITVDIGTP
S
>NP_001365425.1 B4GALT1 [organism=Homo sapiens] [GeneID=2683] [isoform=3] (**SEQ ID NO:39**) 
MRLREPLLSGSAAMPGASLQRACRLLVAVCALHLGVTLVYYLAGRDLSRLPQLVGVSTPLQGGS
NSAAAIGQSSGELRTGGARPPPPLGASSQPRPGGDSSPVVDSGPGPASNLTSVPVPHTTALSLP
ACPEESPLLVGPMLIEFNMPVDLELVAKQNPNVKMGGRYAPRDCVSPHKVAIIIPFRNRQEHLK
YWLYYLHPVLQRQQLDYGIYVINQAGDTIFNRAKLLNVGFQEALKDYDYTCFVFSDVDLIPMND
HNAYRCFSQPRHISVAMDKFGFRLVFRGMSISRPNAVVGRCRMIRHSRDKKNEPNPQRFDRIAH
TKETMLSDGLNSLTYQVLDVQRYPLYTQITVDIGTPS
>NP_001365426.1 B4GALT1 [organism=Homo sapiens] [GeneID=2683] [isoform=4] (**SEQ ID NO:40**)

MRLREPLLSGSAAMPGASLQRACRLLVAVCALHLGVTLVYYLAGRDLSRLPQLVGVSTPLQGGS
NSAAAIGQSSGELRTGGARPPPPLGASSQPRPGGDSSPVVDSGPGPASNLTSVPVPHTTALSLP
ACPEESPLLVGPMLIEFNMPVDLELVAKQNPNVKMGGRYAPRDCVSPHKVAIIIPFRNRQEHLK
YWLYYLHPVLQRQQLDYGIYVINQYEKIRRLLW

**Table 2. Topology of B4GALT1 isoform 1 (SEQ ID NO:37)**

| **Feature** | **AAs** | **Description** | **Length** | **Sequence** | **SE Q ID NO:** |
|---|---|---|---|---|---|
| Topological domain | 1 - 24 | Cytoplasmic | 9 | MRLREPLLSGSAAMPGASLQRACR | **41** |
| Transmembrane | 25 - 44 | Helical; Signal-anchor for type II membrane protein | 17 | LLVAVCALHLGVTLVYYLAG | **42** |
| Topological domain | 45 - 398 | Lumenal | 380 | RDLSRLPQLVGVSTPLQGGSNSAAAIGQ SSGELRTGGARPPPPLGASSQPRPGGDS SPVVDSGPGPASNLTSVPVPHTTALSLP ACPEESPLLVGPMLIEFNMPVDLELVAK QNPNVKMGGRYAPRDCVSPHKVAIIIPF RNRQEHLKYWLYYLHPVLQRQQLDYGIY VINQAGDTIFNRAKLLNVGFQEALKDYD YTCFVFSDVDLIPMNDHNAYRCFSQPRH ISVAMDKFGFSLPYVQYFGGVSALSKQQ FLTINGFPNNYWGWGGEDDDIFNRLVFR GMSISRPNAVVGRCRMIRHS RDKKNEPNPQRFDRIAHTKETMLSDGLN SLTYQVLDVQRYPLYTQITVDIGTPS | **43** |

**Table 3. Binding sites of B4GALT1 isoform 1 (SEQ ID NO:1)**

| **Position(s)** | **Description** | **Reference(s)** |
|---|---|---|
| 250 | Metal binding; Manganese | |
| 310 | Binding site; UDP-alpha-D-galactose | "Structural snapshots of beta-1,4-galactosyltransferase-I along the kinetic pathway." |
| | | Ramakrishnan B., Ramasamy V., Qasba P.K. |
| | | J. Mol. Biol. 357:1619-1633(2006) |
| 343 | Metal binding; Manganese; via tele nitrogen | |
| 355 | Binding site; N-acetyl-D-glucosamine | "Oligosaccharide preferences of beta1,4-galactosyltransferase-I: crystal structures of Met340His mutant of human beta1,4-galactosyltransferase-I with a pentasaccharide and trisaccharides of the N-glycan moiety." |
| | | Ramasamy V., Ramakrishnan B., Boeggeman E., Ratner D.M., Seeberger P.H., Qasba P.K. |
| | | J. Mol. Biol. 353:53-67(2005) |
| | | "Deoxygenated disaccharide analogs as specific inhibitors of beta1-4-galactosyltransferase 1 and selectin-mediated tumor metastasis." |
| | | Brown J.R., Yang F., Sinha A., Ramakrishnan B., Tor Y., Qasba P.K., Esko J.D. |
| | | J. Biol. Chem. 284:4952-4959(2009) |

**Table 4. Post Translational Amino Acid Modifications of B4GALT1 isoform 1 (SEQ ID NO:37)**

| **Feature key** | **Position(s)** | **Description** | **Reference(s)** |
|---|---|---|---|
| Glycosylation | 113 | N-linked (GlcNAc...) asparagine | |
| Disulfide bond | 130 ↔ 172 | | "Oligosaccharide preferences of beta1,4-galactosyltransferase-I: crystal structures of Met340His mutant of human beta1,4-galactosyltransferase-I with a pentasaccharide and trisaccharides of the N-glycan moiety." |
| Disulfide bond | 243 ↔ 262 | | |
| | | | Ramasamy V., Ramakrishnan B., Boeggeman E., Ratner D.M., Seeberger P.H., Qasba P.K. |
| | | | J. Mol. Biol. 353:53-67(2005) |
| | | | "Structural snapshots of beta-1,4-galactosyltransferase-I along the kinetic pathway." |
| | | | Ramakrishnan B., Ramasamy V., Qasba P.K. |
| | | | J. Mol. Biol. 357:1619-1633(2006) |

The soluble form of B4GalT1 derives from the membrane form by proteolytic processing. The cleavage site is at positions 77-78 of B4GALT1 isoform 1 (**SEQ ID NO:37**).

In some embodiments, one or more of the amino acids of the B4GalT1 corresponding to amino acids 113, 130, 172, 243, 250, 262, 310, 343, or 355 of B4GALT1 isoform 1 (**SEQ ID NO:37**) is conserved as compared to (**SEQ ID NO:37**).

Provided herein are enzymatically active portions of, e.g., B4GalT1. In some embodiments, the enzyme is an enzymatically active portion of B4GALT1 isoform 1 (**SEQ ID NO:37**), or an ortholog, mutant, or variant of **SEQ ID NO:37.** In some embodiments, the enzyme is an enzymatically active portion of B4GALT1 isoform 2 (**SEQ ID NO:38**), or an ortholog, mutant, or variant of **SEQ ID NO:38.** In some embodiments, the enzyme is an enzymatically active portion of B4GALT1 isoform 3 (**SEQ ID NO:39**), or an ortholog, mutant, or variant of **SEQ ID NO:39.** In some embodiments, the enzyme is an enzymatically active portion of B4GALT1 isoform 4 (**SEQ ID NO:40**), or an ortholog, mutant, or variant of **SEQ ID NO:40.**

In some embodiments, the enzymatically active portion of B4GalT1 does not comprise a cytoplasmic domain, e.g., **SEQ ID NO:41.** In some embodiments, the enzymatically active portion of B4GalT1 does not comprise a transmembrane domain, e.g., **SEQ ID NO:42.** In some embodiments, the enzymatically active portion of B4GalT1 does not comprise a cytoplasmic domain, e.g., **SEQ ID NO:41** or a transmembrane domain, e.g., **SEQ ID NO:42.**

In some embodiments, the enzymatically active portion of B4GalT1 comprises all or a portion of a luminal domain, e.g., **SEQ ID NO:43,** or an ortholog, mutants, or variants thereof.

In some embodiments, the enzymatically active portion of B4GalT1 comprises amino acids 109-398 of **SEQ ID NO:37,** or an ortholog, mutants, or variants thereof. In some embodiments, the enzymatically active portion of B4GalT1 consists of **SEQ ID NO:37,** or an ortholog, mutant, or variant of **SEQ ID NO:37.**

A suitable functional portion of an B4GalT1 can comprise or consist of an amino acid sequence that is at least 80% (85%, 90%, 95%, 98% or 100%) identical to **SEQ ID NO:43.**

SEQ ID NO:43

GPASNLTSVPVPHTTALSLPACPEESPLLVGPMLIEFNMPVDLELVAKQNPNVKMGGRYAPRDC
VSPHKVAIIIPFRNRQEHLKYWLYYLHPVLQRQQLDYGIYVINQAGDTIFNRAKLLNVGFQEAL
KDYDYTCFVFSDVDLIPMNDHNAYRCFSQPRHISVAMDKFGFSLPYVQYFGGVSALSKQQFLTI
NGFPNNYWGWGGEDDDIFNRLVFRGMSISRPNAVVGRCRMIRHSRDKKNEPNPQRFDRIAHTKE
TMLSDGLNSLTYQVLDVQRYPLYTQITVDIGTPS

ST6Gal1, e.g., human ST6Gal1, as well as orthologs, mutants, and variants thereof, including enzymatically active portions of ST6Gal1, e.g., human ST6Gal1, as well as orthologs, mutants, and variants thereof, and fusion proteins comprising the same, are suitable for use in the methods described herein. ST6GAL1, β-galactoside α-2,6-sialyltransferase 1, transfers sialic acid from CMP-sialic acid to the Galβ1→4GlcNAc structure on glycoproteins, such as asialofetuin and asialo-a1-acid glycoprotein. ST6Gal1 is also called as ST6N or SIAT1. Four alternative transcripts encoding two isoforms of ST6GAL1 (NCBI Gene ID 6480) are described in **Table 1.**

**Table 1. Human ST6GAL1 isoforms**

| Transcript | Length (nt) | Protein | **SEQ ID NO:** | Length (aa) | Isoform |
|---|---|---|---|---|---|
| NM_173216.2 | 4604 | NP_775323.1 | **28** | 406 | a |
| NM_173217.2 | 3947 | NP_775324.1 | **29** | 175 | b |
| NM_003032.3 | 4303 | NP_003023.1 | **28** | 406 | a |
| NM_001353916.2 | 4177 | NP_001340845.1 | **28** | 406 | a |

>NP_001340845.1 (NP_003023.1, NP_775323.1) ST6GAL1 [organism=Homo sapiens] [GeneID=6480] [isoform=a] (**SEQ ID NO:28**) 
MIHTNLKKKFSCCVLVFLLFAVICVWKEKKKGSYYDSFKLQTKEFQVLKSLGKLAMGSDSQSVS
SSSTQDPHRGRQTLGSLRGLAKAKPEASFQVWNKDSSSKNLIPRLQKIWKNYLSMNKYKVSYKG
PGPGIKFSAEALRCHLRDHVNVSMVEVTDFPFNTSEWEGYLPKESIRTKAGPWGRCAVVSSAGS
LKSSQLGREIDDHDAVLRFNGAPTANFQQDVGTKTTIRLMNSQLVTTEKRFLKDSLYNEGILIV
WDPSVYHSDIPKWYQNPDYNFFNNYKTYRKLHPNQPFYILKPQMPWELWDILQEISPEEIQPNP
PSSGMLGIIIMMTLCDQVDIYEFLPSKRKTDVCYYYQKFFDSACTMGAYHPLLYEKNLVKHLNQ
GTDEDIYLLGKATLPGFRTIHC
>NP_775324.1 ST6GAL1 [organism=Homo sapiens] [GeneID=6480] [isoform=b] (**SEQ ID NO:29**)

MNSQLVTTEKRFLKDSLYNEGILIVWDPSVYHSDIPKWYQNPDYNFFNNYKTYRKLHPNQPFYI
LKPQMPWELWDILQEISPEEIQPNPPSSGMLGIIIMMTLCDQVDIYEFLPSKRKTDVCYYYQKF
FDSACTMGAYHPLLYEKNLVKHLNQGTDEDIYLLGKATLPGFRTIHC

**Table 2. Topology of ST6Gal1 isoform a (SEQ ID NO:28)**

| **Feature** | **AAs** | **Description** | **Length** | **Sequence** | **SEQ ID NO:** |
|---|---|---|---|---|---|
| Topological domain | 1 - 9 | Cytoplasmic | 9 | MIHTNLKKK | 34 |
| Transmembrane | 10 - 26 | Helical; Signal-anchor for type II membrane protein | 17 | FSCCVLVFLLFAVICVW | 35 |
| Topological domain | 27 - 406 | Lumenal | 380 | KEKKKGSYYDSFKLQTKEFQVLKSLGK LAMGSDSQSVSSSSTQDPHRGRQTLGS LRGLAKAKPEASFQVWNKDSSSKNLIP RLQKIWKNYLSMNKYKVSYKGPGPGIK FSAEALRCHLRDHVNVSMVEVTDFPFN TSEWEGYLPKESIRTKAGPWGRCAVVS SAGSLKSSQLGREIDDHDAVLRFNGAP TANFQQDVGTKTTIRLMNSQLVTTEKR FLKDSLYNEGILIVWDPSVYHSDIPKW YQNPDYNFFNNYKTYRKLHPNQPFYIL KPQMPWELWDILQEISPEEIQPNPPSS GMLGIIIMMTLCDQVDIYEFLPSKRKT DVCYYYQKFFDSACTMGAYHPLLYEKN LVKHLNQGTDEDIYLLGKATLPGFRTI HC | 36 |

**Table 3. Binding sites of ST6Gal1 isoform a (SEQ ID NO:28)**

| **Position(s)** | **Description** | **Reference(s)** |
|---|---|---|
| 189 | Substrate; via amide nitrogen | "The structure of human alpha-2,6-sialyltransferase reveals the binding mode of complex glycans." |
| 212 | Substrate | |
| 233 | Substrate | |
| 353 | Substrate; via carbonyl oxygen | Kuhn B., Benz J., Greif M., Engel A.M., Sobek H., Rudolph M.G. Acta Crystallogr. D 69:1826-1838(2013) |
| 354 | Substrate | |
| 365 | Substrate | |
| 369 | Substrate | |
| 370 | Substrate | "The structure of human alpha-2,6-sialyltransferase reveals the binding mode of complex glycans." |
| 376 | Substrate | Kuhn B., Benz J., Greif M., Engel A.M., Sobek H., Rudolph M.G. Acta Crystallogr. D 69:1826-1838(2013) |

**Table 4. Post Translational Amino Acid Modifications of ST6Gal1 isoform a (SEQ ID NO:28)**

| **Feature key** | **Position(s)** | **Description** | **Reference(s)** |
|---|---|---|---|
| Disulfide bond | 142 ↔ 406 | | "The structure of human alpha-2,6-sialyltransferase reveals the binding mode of complex glycans." |
| | | | Kuhn B., Benz J., Greif M., Engel A.M., Sobek H., Rudolph M.G. |
| | | | Acta Crystallogr. D 69:1826-1838(2013) |
| Glycosylation | 149 | N-linked (GlcNAc...) asparagine | "Glycoproteomics analysis of human liver tissue by combination of multiple enzyme digestion and hydrazide chemistry." |
| | | | Chen R., Jiang X., Sun D., Han G., Wang F., Ye M., Wang L., Zou H. |
| | | | J. Proteome Res. 8:651-661(2009); and |
| | | | "The structure of human alpha-2,6-sialyltransferase reveals the binding mode of complex glycans." |
| | | | Kuhn B., Benz J., Greif M., Engel A.M., Sobek H., Rudolph M.G. |
| | | | Acta Crystallogr. D 69:1826-1838(2013) |
| Glycosylation | 161 | N-linked (GlcNAc...) asparagine | "Glycoproteomics analysis of human liver tissue by combination of multiple enzyme digestion and hydrazide chemistry." |
| | | | Chen R., Jiang X., Sun D., Han G., Wang F., Ye M., Wang L., Zou H. |
| | | | J. Proteome Res. 8:651-661(2009) |
| Disulfide bond | 184 ↔ 335 | | "The structure of human alpha-2,6-sialyltransferase reveals the binding mode of complex glycans." |
| | | | Kuhn B., Benz J., Greif M., Engel A.M., Sobek H., Rudolph M.G. |
| | | | Acta Crystallogr. D 69:1826-1838(2013) |
| Disulfide bond | 353 ↔ 364 | | "The structure of human alpha-2,6-sialyltransferase reveals the binding mode of complex glycans." |
| | | | Kuhn B., Benz J., Greif M., Engel A.M., Sobek H., Rudolph M.G. |
| | | | Acta Crystallogr. D 69:1826-1838(2013) |
| Modified residue | 369 | Phosphotyrosine | "Quantitative phosphoproteomic analysis of T cell receptor signaling reveals system-wide modulation of protein-protein interactions." |
| | | | Mayya V., Lundgren D.H., Hwang S.-I., Rezaul K., Wu L., Eng J.K., Rodionov V., Han D.K. |
| | | | Sci. Signal. 2:RA46-RA46(2009) |

The soluble form of ST6Gal1 derives from the membrane form by proteolytic processing.

In some embodiments, one or more of the amino acids of the ST6Gal1 corresponding to amino acids 142, 149, 161, 184, 189, 212, 233, 335, 353, 354, 364, 365, 369, 370, 376, or 406 of ST6Gal1 isoform a (**SEQ ID NO:28**) is conserved as compared to **SEQ ID NO:28.**

Also provided herein is an enzymatically active portion of, e.g., ST6Gal1. In some embodiments, the enzyme is an enzymatically active portion of STG6Gal1 isoform a (**SEQ ID NO:28**), or an ortholog, mutant, or variant of **SEQ ID NO:28.** In some embodiments, the enzyme is an enzymatically active portion of STG6Gal1 isoform b (**SEQ ID NO:29**), or an ortholog, mutant, or variant of **SEQ ID NO:29.**

In some embodiments, the enzymatically active portion of ST6Gal1 does not comprise a cytoplasmic domain, e.g., **SEQ ID NO:34.** In some embodiments, the enzymatically active portion of ST6Gal1 does not comprise a transmembrane domain, e.g., **SEQ ID NO:35.** In some embodiments, the enzymatically active portion of ST6Gal1 does not comprise a cytoplasmic domain, e.g., **SEQ ID NO:34** or a transmembrane domain, e.g., **SEQ ID NO:35.**

In some embodiments, the enzymatically active portion of ST6Gal1 comprises all or a portion of a luminal domain, e.g., **SEQ ID NO:36,** or an ortholog, mutants, or variants thereof.

In some embodiments, the enzymatically active portion of ST6Gal1 comprises amino acids 87-406 of **SEQ ID NO:28** (**SEQ ID NO:4**), or an ortholog, mutants, or variants thereof. In some embodiments, the enzymatically active portion of ST6Gal1 consists of **SEQ ID NO:4,** or an ortholog, mutant, or variant of **SEQ ID NO:4.**

A suitable functional portion of an ST6Gal1 can comprise or consist of an amino acid sequence that is at least 80% (85%, 90%, 95%, 98% or 100%) identical to **SEQ ID NO:3** or **SEQ ID NO:4.**

SEQ ID NO:4

AKPEASFQVWNKDSSSKNLIPRLQKIWKNYLSMNKYKVSYKGPGPGIKFSAEALRCHLRDHVNV
SMVEVTDFPFNTSEWEGYLPKESIRTKAGPWGRCAVVSSAGSLKSSQLGREIDDHDAVLRFNGA
PTANFQQDVGTKTTIRLMNSQLVTTEKRFLKDSLYNEGILIVWDPSVYHSDIPKWYQNPDYNFF
NNYKTYRKLHPNQPFYILKPQMPWELWDILQEISPEEIQPNPPSSGMLGIIIMMTLCDQVDIYE
FLPSKRKTDVCYYYQKFFDSACTMGAYHPLLYEKNLVKHLNQGTDEDIYLLGKATLPGFRTIHC

### Variants

In some embodiments, the enzyme(s) described herein are at least 80%, e.g., at least 85%, 90%, 95%, 98%, or 100% identical to the amino acid sequence of an exemplary sequence (e.g., as provided herein), e.g., have differences at up to 1%, 2%, 5%, 10%, 15%, or 20% of the residues of the exemplary sequence replaced, e.g., with conservative mutations, e.g., including or in addition to the mutations described herein. In preferred embodiments, the variant retains desired activity of the parent, e.g., β-galactoside α-2,6-sialyltransferase activity or β-1,4-galactosyltransferase activity.

To determine the percent identity of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). The length of a reference sequence aligned for comparison purposes is at least 80% of the length of the reference sequence, and in some embodiments is at least 90% or 100%. The nucleotides at corresponding amino acid positions or nucleotide positions are then compared. When a position in the first sequence is occupied by the same nucleotide as the corresponding position in the second sequence, then the molecules are identical at that position (as used herein nucleic acid "identity" is equivalent to nucleic acid "homology"). The percent identity between the two sequences is a function of the number of identical positions shared by the sequences, taking into account the number of gaps, and the length of each gap, which need to be introduced for optimal alignment of the two sequences.

Percent identity between a subject polypeptide or nucleic acid sequence (i.e. a query) and a second polypeptide or nucleic acid sequence (i.e. target) is determined in various ways that are within the skill in the art, for instance, using publicly available computer software such as Smith Waterman Alignment (Smith, T. F. and M. S. Waterman (1981) J Mol Biol 147:195-7); "BestFit" (Smith and Waterman, Advances in Applied Mathematics, 482-489 (1981)) as incorporated into GeneMatcher Plus^{™}, Schwarz and Dayhof (1979) Atlas of Protein Sequence and Structure, Dayhof, M.O., Ed, pp 353-358; BLAST program (Basic Local Alignment Search Tool; (Altschul, S. F., W. Gish, et al. (1990) J Mol Biol 215: 403-10), BLAST-2, BLAST-P, BLAST-N, BLAST-X, WU-BLAST-2, ALIGN, ALIGN-2, CLUSTAL, or Megalign (DNASTAR) software. In addition, those skilled in the art can determine appropriate parameters for measuring alignment, including any algorithms needed to achieve maximal alignment over the length of the sequences being compared. In general, for target proteins or nucleic acids, the length of comparison can be any length, up to and including full length of the target (e.g., 5%, 10%, 20%, 30%, 40%, 50%, 60%, 70%, 80%, 90%, 95%, or 100%). For the purposes of the present disclosure, percent identity is relative to the full length of the query sequence.

For purposes of the present disclosure, the comparison of sequences and determination of percent identity between two sequences can be accomplished using a Blossum 62 scoring matrix with a gap penalty of 12, a gap extend penalty of 4, and a frameshift gap penalty of 5.

Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine.

### Fusion Proteins

Also provided herein are fusion protein(s) comprising enzyme(s) or portions thereof as described herein.

In one embodiment, the fusion protein comprises a signal sequence. In some embodiments, the signal sequence is about 15 to about 20 amino acid, e.g., about 15, 16, 17, 18, 19, or 20 amino acids long. In some embodiments, the signal sequence comprises a hydrophobic core region (h-region) flanked by an n-region and a c-region. In some embodiments, the c-region comprises a signal peptidase consensus cleavage site.

In some embodiments, signal sequence is an N-terminal signal sequence.

In some embodiments, the signal sequence is an azurocidin signal sequence. In some embodiments, the azurocidin signal sequence comprises or consists of MTRLTVLALLAGLLASSRA (**SEQ ID NO:30**). In some embodiments, the signal sequence is a serum albumin signal sequence. In some embodiments, the serum albumin signal sequence comprises or consists of MKWVTFISLLFLFSSAYS (**SEQ ID NO:46**). In some embodiments, the signal sequence is an immunoglobulin heavy chain signal sequence. In some embodiments, the immunoglobulin heavy chain signal sequence comprises or consists of MDWTWRVFCLLAVTPGAHP (**SEQ ID NO:47**). In some embodiments, the signal sequence is an immunoglobulin light chain signal sequence. In some embodiments, the immunoglobulin light chain signal sequence comprises or consists of MDWTWRVFCLLAVTPGAHP (**SEQ ID NO:48**).

In some embodiments, the signal sequence is a cystatin-S signal sequence. In some embodiments, the cystatin-S signal sequence comprises or consists of MARPLCTLLLLMATLAGALA (**SEQ ID NO:49**). In some embodiments, the signal sequence is an IgKappa signal sequence. In some embodiments, the IgKappa signal sequence comprises or consists of MDMRAPAGIFGFLLVLFPGYRS (**SEQ ID NO:50**). In some embodiments, the signal sequence is a trypsonigen 2 signal sequence. In some embodiments, the trysonigen 2 signal sequence comprises or consists of MRSLVFVLLIGAAFA (**SEQ ID NO:51**). In some embodiments, the signal sequence is potassium channel blocker signal sequence. In some embodiments, the potassium channel blocker sequence comprises or consists of MSRLFVFILIALFLSAIIDVMS (**SEQ ID NO:52**).

In some embodiments, the signal sequence is an alpha conotoxin Ip 1.3 signal sequence. In some embodiments, the alpha conotoxin Ip1.3 signal sequence comprises or consists of MGMRMMFIMFMLVVLATTVVS (**SEQ ID NO:53**). In some embodiments, the signal sequence is an alfa-galactosidase signal sequence. In some embodiments, the alfa-galactosidase signal sequence comprises or consists of MRAFLFLTACISLPGVFG (**SEQ ID NO:54**). In some embodiments, the signal sequence is a cellulase signal sequence. In some embodiments, the cellulase signal sequence comprises or consists of MKFQSTLLLAAAAGSALA (**SEQ ID NO:55**). In some embodiments, signal sequence is an aspartic proteinase nepenthesin-1 signal sequence. In some embodiments, the aspartic proteinase nepenthesin-1 signal sequence comprises or consists of MASSLYSFLLALSIVYIFVAPTHS (**SEQ ID NO:56**). In some embodiments, the signal sequence is an acid chitinase signal sequence. In some embodiments, the acid chitinase signal sequence comprises or consists of MKTHYSSAILPILTLFVFLSINPSHG (**SEQ ID NO:57**). In some embodiments, the signal sequence is a K28 prepro-toxin signal sequence. In some embodiments, the K28 prepro-toxin signal sequence comprises or consists of MESVSSLFNIFSTIMVNYKSLVLALLSVSNLKYARG (**SEQ ID NO:58**). In some embodiments, the signal sequence is a killer toxin zygocin precursor signal sequence. In some embodiments, the killer toxin zygocin precursor signal sequence comprises or consists of MKAAQILTASIVSLLPIYTSA (**SEQ ID NO:59**). In some embodiments, the signal sequence is a cholera toxin signal sequence. In some embodiments, the cholera toxin signal sequence comprises or consists of MIKLKFGVFFTVLLSSAYA (**SEQ ID NO:60**). In some embodiments, the signal sequence is a human growth hormone signal sequence. In some embodiments, the human growth hormone signal sequence comprises or consists of MATGSRTSLLLAFGLLCLPWLQEGSA (**SEQ ID NO:61**)

In some embodiments, the fusion protein comprises one or more affinity tag(s). In some embodiments, the purification tag is selected from the group consisting of polyhistidine, glutathione S-transferase (GST), maltose-binding protein (MBP), chitin binding protein, a streptavidin tag (e.g., Strep-Tag^{®}, e.g., Trp-Ser-His-Pro-Gln-Phe-Glu-Lys (**SEQ ID NO:31**)), FLAG-tag (e.g., DYKDDDDK (**SEQ ID NO:32**)), a biotin tag (e.g., AviTag^{™}) and combinations thereof.

In some embodiments, the affinity tag is situated towards the N-terminal side of the enzyme or portion thereof. In some embodiments, the affinity tag is N-terminal.

In some embodiments, the affinity tag is situated towards the C-terminal side of the enzyme or portion thereof. In some embodiments, the affinity tag is C-terminal.

In some embodiments, the affinity tag is a polyhistidine tag. In some embodiments, the polyhistidine tag is selected from the group consisting of HHHH (**SEQ ID NO:11**), HHHHH (**SEQ ID NO:12**), HHHHHH, (**SEQ ID NO:13**), HHHHHHH (**SEQ ID NO:14**), HHHHHHHH (**SEQ ID NO:15**), HHHHHHHHH (**SEQ ID NO:16**), and HHHHHHHHHH (**SEQ ID NO:17**). In some embodiments, the polyhistidine tag is a hexahistidine tag (e.g., HHHHHH (**SEQ ID NO:13**)).

In some embodiments, the fusion protein comprises or consists of **SEQ ID NO:43, SEQ ID NO:44,** or **SEQ ID NO:45.**
**SEQ ID NO:44** 
GPASNLTSVPVPHTTALSLPACPEESPLLVGPMLIEFNMPVDLELVAKQNPNVKMGGRYAPRDC
VSPHKVAIIIPFRNRQEHLKYWLYYLHPVLQRQQLDYGIYVINQAGDTIFNRAKLLNVGFQEAL
KDYDYTCFVFSDVDLIPMNDHNAYRCFSQPRHISVAMDKFGFSLPYVQYFGGVSALSKQQFLTI
NGFPNNYWGWGGEDDDIFNRLVFRGMSISRPNAVVGRCRMIRHSRDKKNEPNPQRFDRIAHTKE
TMLSDGLNSLTYQVLDVQRYPLYTQITVDIGTPSPRD
**SEQ ID NO:45**

gssplldmGPASNLTSVPVPHTTALSLPACPEESPLLVGPMLIEFNMPVDLELVAKQNPNVKMG
GRYAPRDCVSPHKVAIIIPFRNRQEHLKYWLYYLHPVLQRQQLDYGIYVINQAGDTIFNRAKLL
NVGFQEALKDYDYTCFVFSDVDLIPMNDHNAYRCFSQPRHISVAMDKFGFSLPYVQYFGGVSAL
SKQQFLTINGFPNNYWGWGGEDDDIFNRLVFRGMSISRPNAVVGRCRMIRHSRDKKNEPNPQRF
DRIAHTKETMLSDGLNSLTYQVLDVQRYPLYTQITVDIGTPSprdhhhhhh

In some embodiments, the fusion protein comprises or consists of **SEQ ID NO:3** or **SEQ ID NO:5.**
**SEQ ID NO:3** 
gsspl1dmlehhhhhhhhmAKPEASFQVWNKDSSSKNLIPRLQKIWKNYLSMNKYKVSYKGPGP
GIKFSAEALRCHLRDHVNVSMVEVTDFPFNTSEWEGYLPKESIRTKAGPWGRCAVVSSAGSLKS
SQLGREIDDHDAVLRFNGAPTANFQQDVGTKTTIRLMNSQLVTTEKRFLKDSLYNEGILIVWDP
SVYHSDIPKWYQNPDYNFFNNYKTYRKLHPNQPFYILKPQMPWELWDILQEISPEEIQPNPPSS
GMLGIIIMMTLCDQVDIYEFLPSKRKTDVCYYYQKFFDSACTMGAYHPLLYEKNLVKHLNQGTD
EDIYLLGKATLPGFRTIHC
**SEQ ID NO:5**

hhhhhhhhmAKPEASFQVWNKDSSSKNLIPRLQKIWKNYLSMNKYKVSYKGPGPGIKFSAEALR
CHLRDHVNVSMVEVTDFPFNTSEWEGYLPKESIRTKAGPWGRCAVVSSAGSLKSSQLGREIDDH
DAVLRFNGAPTANFQQDVGTKTTIRLMNSQLVTTEKRFLKDSLYNEGILIVWDPSVYHSDIPKW
YQNPDYNFFNNYKTYRKLHPNQPFYILKPQMPWELWDILQEISPEEIQPNPPSSGMLGIIIMMT
LCDQVDIYEFLPSKRKTDVCYYYQKFFDSACTMGAYHPLLYEKNLVKHLNQGTDEDIYLLGKAT
LPGFRTIHC

### Expression Systems

To use the enzyme(s) and/or fusion protein(s) described herein, it may be desirable to express them from a nucleic acid that encodes them. This can be performed in a variety of ways. For example, the nucleic acid encoding the enzyme(s) and/or fusion protein(s) can be cloned into an intermediate vector for transformation into prokaryotic or eukaryotic cells for replication and/or expression. Intermediate vectors are typically prokaryote vectors, e.g., plasmids, or shuttle vectors, or insect vectors, for storage or manipulation of the nucleic acid encoding the enzyme(s) and/or fusion protein(s). The nucleic acid encoding the enzyme(s) and/or fusion protein(s) can also be cloned into an expression vector, for administration to a plant cell, animal cell, preferably a mammalian cell or a human cell, fungal cell, bacterial cell, or protozoan cell.

To obtain expression, a sequence encoding the enzyme(s) and/or fusion protein(s) is typically subcloned into an expression vector that contains a promoter to direct transcription. Suitable bacterial and eukaryotic promoters are well known in the art and described, e.g., in Sambrook et al., Molecular Cloning, A Laboratory Manual (3d ed. 2001); Kriegler, Gene Transfer and Expression: A Laboratory Manual (1990); and Current Protocols in Molecular Biology (Ausubel et al., eds., 2010). Bacterial expression systems for expressing engineered protein are available in, e.g., *E. coli*, *Bacillus* sp., and *Salmonella* (Palva et al., 1983, Gene 22:229-235). Kits for such expression systems are commercially available. Eukaryotic expression systems for mammalian cells, yeast, and insect cells are well known in the art and are also commercially available.

The promoter used to direct expression of a nucleic acid depends on the particular application. For example, a strong constitutive promoter is typically used for expression and purification of fusion proteins.

In some embodiments, the promoter is selected from the group consisting of human cytomegalovirus (CMV), EF-1 α (EF1A), elongation factor 1α short (EFS), CMV enhancer chicken β-Actin promoter and rabbit β-Globin splice acceptor site (CAG), hybrid CBA (CBh), spleen focus-forming virus (SFFV), murine stem cell virus (MSCV), simian virus 40 (SV40), mouse phosphoglycerate kinase 1 (mPGK), human phosphoglycerate kinase 1 (hPGK), and ubiquitin C (UBC) promoters. In some embodiments, the promoter is a human cytomegalovirus promoter (CMV).

In addition to the promoter, the expression vector typically contains a transcription unit or expression cassette that contains all the additional elements required for the expression of the nucleic acid in host cells, either prokaryotic or eukaryotic. A typical expression cassette thus contains a promoter operably linked, e.g., to the nucleic acid sequence encoding the enzyme(s) and/or fusion protein(s) and any signals required, e.g., for efficient polyadenylation of the transcript, transcriptional termination, ribosome binding sites, or translation termination. Additional elements of the cassette may include, e.g., enhancers, and heterologous spliced intronic signals.

In some embodiments, the expression vector comprises a woodchuck hepatitis virus posttranscriptional regulatory element (WPRE). *See, e.g.,* Zufferey et al., "Woodchuck Hepatitis Virus Posttranscriptional Regulatory Element Enhances Expression of Transgenes Delivered by Retroviral Vectors," Journal of Virology 73(4):2886-92 (1999).

The particular expression vector used to transport the genetic information into the cell is selected with regard to the intended use of the enzyme(s) and/or fusion protein(s), e.g., expression in plants, animals, bacteria, fungus, protozoa, etc.

Standard transfection methods are used to produce bacterial, mammalian, yeast or insect cell lines that express large quantities of protein, which are then purified using standard techniques (see, e.g., Colley et al., 1989, J. Biol. Chem., 264:17619-22; Guide to Protein Purification, in Methods in Enzymology, vol. 182 (Deutscher, ed., 1990)). Transformation of eukaryotic and prokaryotic cells are performed according to standard techniques (see, e.g., Morrison, 1977, J. Bacteriol. 132:349-351; Clark-Curtiss & Curtiss, Methods in Enzymology 101:347-362 (Wu et al., eds, 1983).

Any of the known procedures for introducing foreign nucleotide sequences into host cells may be used. These include the use of calcium phosphate transfection, polybrene, protoplast fusion, electroporation, nucleofection, liposomes, microinjection, naked DNA, plasmid vectors, viral vectors, both episomal and integrative, and any of the other well-known methods for introducing cloned genomic DNA, cDNA, synthetic DNA or other foreign genetic material into a host cell (see, e.g., Sambrook et al., supra). It is only necessary that the particular genetic engineering procedure used be capable of successfully introducing at least one gene into the host cell capable of expressing the enzyme(s) and/or fusion protein(s).

In some embodiments, the host cells are stably transformed.

In some embodiments, the host cells are grow under non-hypoxic conditions.

The enzyme(s) and/or fusion protein(s) described herein can be produced by any protein production system known in the art, such as host cell based expression systems, synthetic biology platforms, or cell-free protein production platforms. In some embodiments, the protein production system is capable of post-translational modification(s), including, but not limited to one or more of glycosylation, e.g., N-glycosylated proteins, disulfide bond formation, and tyrosine phosphorylation. See, e.g., Boh and Ng, "Impact of Host Cell Line Choice on Glycan Profile," Critical Reviews in Biotechnology 38(6):851-67 (2018).

In some embodiments, the host cell is a mammalian host cell. In some embodiments, the mammalian cell is selected from the group consisting of Chinese hamster ovary (CHO) cells, baby hamster kidney (BHK) cells, NS0 myeloma cells, Sp2/0 hybridoma mouse cells, human embryonic kidney cells (HEK), HT-1080 human cells, and derivatives thereof.

In some embodiments, the host cell is a non-human mammalian host cell. In some embodiments, the non-human mammalian host cell is selected from CHO cells, BHK-21 cells, murine NS0 myeloma cells, Sp2/0 hybridoma cells, and derivatives thereof.

In some embodiments, the host cell is a human mammalian host cell. In some embodiments, the human cell is selected from the group consisting of HEK, PER.C6, CEVEC's amniocyte production (CAP), AGE1.HM, HKB-11, HT-1080 cells, and derivatives thereof.

In some embodiments, the host cell is a human embryonic kidney cell (HEK, ATCC^{®} CRL-1573^{™}) or derivative thereof.

In some embodiments, the HEK cell expresses a temperature sensitive allele of the SV40 T antigen. In some embodiments, the HEK cell is resistant against the Ricin toxin after ethymethanesulfonate (EMS) mutagenesis and lack N-acetylglucosaminyltransferase I activity, e.g., encoded by the MGAT1 gene. In some embodiments, the HEK cell predominantly modifies glycoproteins with the Man5GlcNAc2 N-glycan. In some embodiments, the HEK cell expresses the tetR repressor, enabling tetracycline-inducible protein expression.

In some embodiments, the HEK derivative is selected from the group consisting of HEK293, HEK293T (293tsA1609neo, ATCC^{®} CRL-3216^{™}), HEK293T/17 (ATCC^{®} CRL-11268^{™}), HEK293T/17 SF (ATCC^{®} ACS-4500^{™}), HEK293S, HEK293SG, HEK293FTM, HEK293SGGD, HEK293FTM, HEK293E, and HKB-11.

Synthetic biology platforms, such as those described in Kightlinger et al., "Synthetic Glycobiology: Parts, Systems, and Applications," ACS Synth. Biol. 9:1534-62 (2020) are also suitable for producing the enzyme(s) and/or fusion protein(s) described herein.

Also provided herein are vectors and cells comprising the vectors, as well as kits comprising the proteins and nucleic acids described herein, e.g., for use in a method described herein.

### EXAMPLES

The invention is further described in the following examples, which do not limit the scope of the invention described in the claims.

### Example 1: Hypersialylated IgG Preparation

IgG in which more than 60% of the overall branched glycans are disialylated can be prepared as follows.

Briefly, a mixture of IgG antibodies is exposed to a sequential enzymatic reaction using β1,4 galactosyltransferase 1 (B4-GalT) and α2,6-sialyltransferase (ST6-Gal1) enzymes. The B4-GalT does not need to be removed from the reaction before addition of ST6-Gal1 and no partial or complete purification of the product is needed between the enzymatic reactions.

The galactosyltransferase enzyme selectively adds galactose residues to pre-existing asparagine-linked glycans. The resulting galactosylated glycans serve as substrates to the sialic acid transferase enzyme which selectively adds sialic acid residues to cap the asparagine-linked glycan structures attached to. Thus, the overall sialylation reaction employed two sugar nucleotides (uridine 5'-diphosphogalactose (UDPGal) and cytidine-5'-monophospho-N-acetylneuraminic acid (CMP-NANA)). The latter is replenished periodically to increase disialylated product relative to monosialylated product. The reaction includes the co-factor manganese chloride.

A representative example of the IgG-Fc glycan profile for such a reaction starting with IVIg and the reaction product is shown in **FIG. 4****.** In **FIG. 4****,** on the left is a schematic representation of enzymatic sialylation reaction to transform IgG to hsIgG; on the right is the IgG Fc glycan profile for the starting IVIg and hsIgG. In this study, glycan profiles for the different IgG subclasses are derived via glycopeptide mass spectrometry analysis. The peptide sequences used to quantify glycopeptides for different IgG subclasses were: IgG1 = EEQYNSTYR (**SEQ ID NO:7**), IgG2/3 EEQFNSTFR (**SEQ ID NO:8**), IgG3/4 EEQYNSTFR (**SEQ ID NO:9**) and EEQFNSTYR (**SEQ ID NO:10**).

The glycan data is shown per IgG subclass. Glycans from IgG3 and IgG4 subclasses cannot be quantified separately. As shown, for IVIg the sum of all the nonsialylated glycans is more than 80% and the sum of all sialylated glycans is < 20%. For the reaction product, the sum for all nonsialylated glycans is < 20% and the sum for all sialylated glycans is more than 80%. Nomenclature for different glycans listed in the glycoprofile use the Oxford notation for N linked glycans.

### Example 2: Alternative Sialylation Condition

Alternative suitable reaction conditions for galactosylation and sialylation to create hsIgG in, e.g., 50 mM BIS-TRIS pH 6.9 include: galactosylation of IgG antibodies (e.g., pooled IgG antibodies, pooled immunoglobulins or IVIg) as follows: 7.4 mM MnCl₂; 38 µmol UDP-Gal/g IgG antibody; and 7.5 units B4GalT/g IgG antibody with 16-24 hours of incubation at 37°C followed by sialylation in 7.4 mM MnCl₂; 220 µmol CMP-NANA/g IgG antibody (added twice: once at the start of the reaction and again after 9-10 hrs); and 15 units ST6-Gal1/g IgG antibody with 30-33 hours of incubation at 37°. The reaction can be carried out by adding the ST6-Gal1 and CMP-NANA to the galactosylation reaction. Alternatively, all of the reactants can be combined at the outset and the CMP-NANA supplemented.

### Example 3: Production of ST6Gal

A fusion protein that includes an enzymatically active portion of ST6Gal was designed for high level expression in HEK cells and ease of purification. **SEQ ID NO:6** is the immature fusion protein which includes a portion of human ST6Gal (**SEQ ID NO:4**), a 6 HIS tag, a signal sequence from azurocidin (MTRLTVLALL AGLLASSRAGSSPLLD (**SEQ ID NO:31**); 19 aa is signal is underlined) and amino acids resulting from the cloning process. **SEQ ID NO:3** is the secreted form, and **SEQ ID NO:5** includes the 6 HIS tag and the ST6GalT portion.
**SEQ ID NO: 6** 
MTRLTVLALLAGLLASSRAGSSPLLDMLEHHHHHHHHMAKPEASFQVWNKDSSSKNLI
PRLQKIWKNYLSMNKYKVSYKGPGPGIKFSAEALRCHLRDHVNVSMVEVTDFPFNTSE
WEGYLPKESIRTKAGPWGRCAVVSSGSLKSSQLGREIDDHDAVLRFNGAPTANFQQDV
GTKTTIRLMNSQLVTTEKRFLKDSLYNEGILIVWDPSVYHSDIPKWYQNPDYNFFNNYK
TYRKLHPNQPFYILKPQMPWELWDILQEISPEEIQPNPPSSGMLGIIIMMTLCDQVDIYEFL
PSKRKTDVCYYYQKFFDSACTMGAYHPLLYEKNLVKHLNQGTDEDIYLLGKATLPGFR
TIHC
**SEQ ID NO: 3** 
gssplldmlehhhhhhhhmAKPEASFQVWNKDSSSKNLIPRLQKIWKNYLSMNKYKVSYKGPG
PGIKFSAEALRCHLRDHVNVSMVEVTDFPFNTSEWEGYLPKESIRTKAGPWGRCAVVSS
AGSLKSSQLGREIDDHDAVLRFNGAPTANFQQDVGTKTTIRLMNSQLVTTEKRFLKDSL
YNEGILIVWDPSVYHSDIPKWYQNPDYNFFNNYKTYRKLHPNQPFYILKPQMPWELWDI
LQEISPEEIQPNPPSSGMLGIIIMMTLCDQVDIYEFLPSKRKTDVCYYYQKFFDSACTMGA
YHPLLYEKNLVKHLNQGTDEDIYLLGKATLPGFRTIHC
**SEQ ID NO: 4** 
AKPEASFQVWNKDSSSKNLIPRLQKIWKNYLSMNKYKVSYKGPGPGIKFSAEALRCHLR
DHVNVSMVEVTDFPFNTSEWEGYLPKESIRTKAGPWGRCAVVSSAGSLKSSQLGREIDD
HDAVLRFNGAPTANFQQDVGTKTTIRLMNSQLVTTEKRFLKDSLYNEGILIVWDPSVYH
SDIPKWYQNPDYNFFNNYKTYRKLHPNQPFYILKPQMPWELWDILQEISPEEIQPNPPSS
GMLGIIIMMTLCDQVDIYEFLPSKRKTDVCYYYQKFFDSACTMGAYHPLLYEKNLVKH
LNQGTDEDIYLLGKATLPGFRTIHC
**SEQ ID NO: 5**

hhhhhhhhmAKPEASFQVWNKDSSSKNLIPRLQKIWKNYLSMNKYKVSYKGPGPGIKFSA
EALRCHLRDHVNVSMVEVTDFPFNTSEWEGYLPKESIRTKAGPWGRCAVVSSAGSLKS
SQLGREIDDHDAVLRFNGAPTANFQQDVGTKTTIRLMNSQLVTTEKRFLKDSLYNEGILI
VWDPSVYHSDIPKWYQNPDYNFFNNYKTYRKLHPNQPFYILKPQMPWELWDILQEISPE
EIQPNPPSSGMLGIIIMMTLCDQVDIYEFLPSKRKTDVCYYYQKFFDSACTMGAYHPLLY
EKNLVKHLNQGTDEDIYLLGKATLPGFRTIHC

HEK293 cells (Expi293F^{®}cells; Life Technologies) were stably transfected with a vector expressing a polypeptide having SEQ ID NO: 6 under the control of a CMV promotor. To produce ST6GalT fusion protein, the stably transfected, and clonally selected cells were counted and seeded on Day 0 at a cell density of 0.4E6 cells/mL, grown at 37°C, 5% CO2, 130-150rpm. On Day 4, a 10% glucose/media feed was added to the cells. Growth was monitored by daily. On Day 7 cell supernatants were harvested, sterile filtered through a 0.45 micron filter and then a 0.2 micron filter.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

Also disclosed are the following clauses:
1. A fusion protein comprising:
   an N-terminal signal sequence; and
   an enzymatically active portion of human Alpha-2,6-sialyltransferase 1 (ST6Gal1).
2. The fusion protein of clause 1, wherein the enzymatically active portion of ST6Gal1 comprises **SEQ ID NO:4.**
3. The fusion protein of clause 2, wherein the enzymatically active portion of ST6Gal1 consists of **SEQ ID NO:4.**
4. The fusion protein of any one of clauses 1-3, wherein the signal sequence is an N-terminal azurocidin signal sequence.
5. The fusion protein of clause 4, wherein the azurocidin signal sequence comprises MTRLTVLALLAGLLASSRA (**SEQ ID NO:30**).
6. The fusion protein of clause 4, wherein the azurocidin signal sequence consists of MTRLTVLALLAGLLASSRA (**SEQ ID NO:30**).
7. The fusion protein of any one of clauses 1-6, further comprising an affinity tag.
8. The fusion protein of clause 7, wherein the affinity tag is selected from the group consisting of polyhistidine, glutathione S-transferase (GST), maltose-binding protein (MBP), chitin binding protein, a streptavidin tag (e.g., Trp-Ser-His-Pro-Gln-Phe-Glu-Lys (**SEQ ID NO:31**)), FLAG-tag (e.g., DYKDDDDK (**SEQ ID NO:32**)), a biotin tag, and combinations thereof.
9. The fusion protein of clause 8, wherein the polyhistidine tag is selected from the group consisting of HHHH (**SEQ ID NO:11**), HHHHH (**SEQ ID NO:12**), HHHHHH, (**SEQ ID NO:13**), HHHHHHH (**SEQ ID NO:14**), HHHHHHHH (**SEQ ID NO:15**), HHHHHHHHH (**SEQ ID NO:16**), and HHHHHHHHHH (**SEQ ID NO:17**).
10. The fusion protein of any one of clauses 7-9, wherein the affinity tag is situated towards the N-terminal side of the enzymatically active portion of ST6Gal1.
11. The fusion protein of clause 1, wherein the N-terminal signal sequence comprises MTRLTVLALLAGLLASSRA (**SEQ ID NO:30**), and the enzymatically active portion of ST6Gal1 comprises **SEQ ID NO:4.**
12. The fusion protein of clause 11, further comprising a hexahistidine tag.
13. The fusion protein of clause 12, wherein the hexahistidine tag is between the N-terminal signal sequence and the enzymatically active portion of ST6Gal1.
14. The fusion protein of clause 13, consisting of **SEQ ID NO:6.**
15. A nucleic acid molecule encoding the fusion protein of any one of clauses 1-14.
16. A vector comprising the nucleic acid molecule of clause 15.
17. The vector of clause 16 further comprising a promoter operably linked to the nucleic acid encoding the fusion protein.
18. The vector of clause 16, wherein the promoter is a cytomegalovirus (CMV) promoter.
19. A host cell stably transformed the vector of clause 16.
20. The host cell of clause 17, wherein the cell is a human embryonic kidney (HEK) cell, or derivative thereof.
21. The host cell of clause 20, wherein the cell is the HEK derivative HEK293.
22. A method for producing a polypeptide comprising:
   culturing the host cell of any one of clauses 19-21 in a culture medium under conditions permissive for expression of the fusion protein; and
   isolating the fusion protein from the culture medium.
23. A fusion protein comprising:
   an N-terminal signal sequence; and
   an enzymatically active portion of human beta-1,4-galactosyltransferase (B4GalT1).
24. The fusion protein of clause 23, wherein the enzymatically active portion of B4GalT1 comprises **SEQ ID NO:43.**
25. The fusion protein of clause 23, wherein the enzymatically active portion of B4GalT1 consists of **SEQ ID NO:43.**
26. The fusion protein of any one of clauses 23-25, wherein the signal sequence is an N-terminal azurocidin signal sequence.
27. The fusion protein of clause 23, wherein the azurocidin signal sequence comprises MTRLTVLALLAGLLASSRA (**SEQ ID NO:30**).
28. The fusion protein of clause 23, wherein the azurocidin signal sequence consists of MTRLTVLALLAGLLASSRA (**SEQ ID NO:30**).
29. The fusion protein of any one of clauses 23-28, further comprising an affinity tag.
30. The fusion protein of clause 29, wherein the affinity tag is selected from the group consisting of polyhistidine, glutathione S-transferase (GST), maltose-binding protein (MBP), chitin binding protein, a streptavidin tag (e.g., Trp-Ser-His-Pro-Gln-Phe-Glu-Lys (**SEQ ID NO:31**)), FLAG-tag (e.g., DYKDDDDK (**SEQ ID NO:32**)), a biotin tag, and combinations thereof.
31. The fusion protein of clause 30, wherein the polyhistidine tag is selected from the group consisting of HHHH (**SEQ ID NO:11**), HHHHH (**SEQ ID NO:12**), HHHHHH, (**SEQ ID NO:13**), HHHHHHH (**SEQ ID NO:14**), HHHHHHHH (**SEQ ID NO:15**), HHHHHHHHH (**SEQ ID NO:16**), and HHHHHHHHHH (**SEQ ID NO:17**).
32. The fusion protein of any one of clauses 23-31, wherein the affinity tag is situated towards the C-terminal side of the enzymatically active portion of B4GalT1.
33. The fusion protein of clause 23, wherein the N-terminal signal sequence comprises MTRLTVLALLAGLLASSRA (**SEQ ID NO:30**), and the enzymatically active portion of B4GalT1comprises **SEQ ID NO:43.**
34. The fusion protein of clause 33, further comprising a septahistidine tag.
35. The fusion protein of clause 34, wherein the septahistidine tag is C-terminal.
36. The fusion protein of clause 35, consisting of **SEQ ID NO:45.**
37. A nucleic acid molecule encoding the fusion protein of any one of clauses 23-36.
38. A vector comprising the nucleic acid molecule of clause 37.
39. The vector of clause 38 further comprising a promoter operably linked to the nucleic acid encoding the fusion protein.
40. The vector of clause 39, wherein the promoter is a cytomegalovirus (CMV) promoter.
41. A host cell stably transformed with the vector of clause 16.
42. The host cell of clause 41, wherein the cell is a human embryonic kidney (HEK) cell, or derivative thereof.
43. The host cell of clause 42, wherein the cell is the HEK derivative HEK293.
44. A method for producing a polypeptide comprising:
   culturing the host cell of any one of clauses 41-43 in a culture medium under conditions permissive for expression of the fusion protein; and
   isolating the fusion protein from the culture medium.
45. A method for sialyating immunoglobulin G (IgG) antibodies, the method comprising:
   a) providing a composition comprising IgG antibodies;
   b) exposing the composition to a β1,4-galactosyltransferase 1 and an enzymatically active portion of ST6Gal1 comprising **SEQ ID NO:4** in the presence of UDP-Gal and CMP-NANA, thereby producing a composition comprising sialyated IgG (sIgG).
46. A method for sialyating immunoglobulin G (IgG) antibodies, the method comprising:
   a) providing a composition comprising IgG antibodies;
   b) exposing the IgG antibodies to a β1,4-galactosyltransferase 1 in the presence of UDP-Gal, thereby producing a composition comprising galactosylated IgG antibodies; and
   c) exposing the composition comprising galactosylated IgG antibodies to an enzymatically active portion of ST6Gal1 comprising **SEQ ID NO:4** in the presence of CMP-NANA, thereby producing a composition comprising sialyated IgG (sIgG).
47. The method of clause 46, wherein neither the composition comprising galactosylated IgG antibodies is not purified prior to step (c).
48. The method of any one of clauses 45-47, further comprising supplementing one or more of the compositions with CMP-NANA.
49. The method of any one of clauses 45-48, where the mixture of IgG antibodies are selected from the group consisting of IgG1, IgG2, IgG3, IgG4, and combinations thereof.
50. The method of clause 45 or 46, wherein at least 60% of branched glycans on the Fc region of the antibodies in the composition comprising sIgG are di-sialylated.
51. The method of clause 45 or 46, wherein less than 50% of branched glycans on the Fc regions of the antibodies in the composition comprising sIgG are mono-sialylated.

## Claims

1. A method for sialyating immunoglobulin G (IgG) antibodies, the method comprising:
(a) providing a composition comprising IgG antibodies;
(b) exposing the composition to a β1,4-galactosyltransferase 1 (B4GalT1) and an alpha-2,6-sialyltransferase 1 (ST6Gal1) comprising **SEQ ID NO:4** in the presence of uridine 5'-diphosphosegalactose (UDP-Gal) and cytidine 5'-monophospho-N-acetylneuraminic acid (CMP-NANA), thereby producing a composition comprising sialyated IgG (sIgG).

2. A method for sialyating immunoglobulin G (IgG) antibodies, the method comprising:
(a) providing a composition comprising IgG antibodies;
(b) exposing the IgG antibodies to a β1,4-galactosyltransferase 1 (B4GalT1) in the presence of uridine 5'-diphosphosegalactose (UDP-Gal), thereby producing a composition comprising galactosylated IgG antibodies; and
(c) exposing the composition comprising galactosylated IgG antibodies to an alpha-2,6-sialyltransferase 1 (ST6Gal1) comprising **SEQ ID NO:4** in the presence of cytidine 5'-monophospho-N-acetylneuraminic acid (CMP-NANA), thereby producing a composition comprising sialyated IgG (sIgG).

3. The method of claim 2, wherein the composition comprising galactosylated IgG antibodies is not purified prior to step (c).

4. The method of any one of the preceding claims, further comprising supplementing one or more of the compositions with CMP-NANA.

5. The method of any one of the preceding claims, where the mixture of IgG antibodies is selected from the group consisting of IgG1, IgG2, IgG3, IgG4, and combinations thereof.

6. The method of any one of the preceding claims, wherein:
(i) at least 60% of branched glycans on the Fc region of the antibodies in the composition comprising sIgG are di-sialylated; or
(ii) less than 50% of branched glycans on the Fc regions of the antibodies in the composition comprising sIgG are mono-sialylated.

7. The method of any one of the preceding claims, wherein:
(i) the B4GalT1 is a soluble form of B4GalT1 derived from a membrane form by proteolytic processing; and/or
(ii) the ST6Gal1 is a soluble form of ST6Gal1 derived from a membrane form by proteolytic processing.

8. The method of any one of the preceding claims, wherein the B4GalT1 comprises **SEQ ID NO:43** or a sequence that is at least 80% identical to **SEQ ID NO:43.**

9. The method of any one of the preceding claims, wherein:
(a) the B4GalT1 is provided in a fusion protein; and/or
(b) the ST6Gal1 is provided in a fusion protein.

10. The method of any one of the preceding claims, wherein
(a) the B4GalT1 was expressed as a fusion protein; and/or
(b) the ST6Gal1 was expressed as a fusion protein.

11. The method of claim 9 or claim 10, wherein the B4GalT1 fusion protein and/or the ST6Gal1 fusion protein comprises a signal sequence.

12. The method of claim 11, wherein
(a) the signal sequence comprises a hydrophobic core region (h-region) flanked by an N-terminal region (n-region) and a C terminal region (c-region), wherein the c-region comprises a signal peptidase consensus cleavage site;
(b) the signal sequence is a N-terminal signal sequence; and/or
(c) the signal sequence is an azurocidin signal sequence, optionally wherein the azurocidin signal sequence comprises or consists of the amino acid sequence of SEQ ID NO: 30.

13. The method of any one of claims 9-12, wherein the B4GalT1 fusion protein and/or the ST6Gal1 fusion protein comprises an affinity tag,
optionally wherein the affinity tag is selected from the group consisting of a polyhistidine tag, glutathione S-transferase (GST), maltose-binding protein (MBP), chitin binding protein, a streptavidin tag, FLAG-tag, a biotin tag, and combinations thereof.

14. The method of any one of claims 9-13, wherein the B4GalT1 fusion protein comprises **SEQ ID NO:45.**

15. The method of any one of claims 9-14, wherein the ST6Gal1 fusion protein comprises the amino acid sequence of **SEQ ID NO:6.**
